# EUROPEAN PATENT SPECIFICATION

(11) **EP 0 724 410 B1**
(45) Date of publication and mention of the grant of the patent: **14.06.2000**
(21) Application number: 95929894.4
(22) Date of filing: 19.08.1995
(51) Int. Cl.: A61B 17/64

(54) **EXTERNAL TROCHANTER SPLINT**
ÄUSSERER FIXATEUR FÜR DEN TROCHANTER
ATTELLE EXTERIEURE DESTINEE AU TROCHANTER

(30) Priority: 23.08.1994 IT VR940075
(43) Date of publication of application: 07.08.1996
(73) Proprietor: ORTHOFIX S.r.l., 37012 Bussolengo (VR) (IT)
(72) Inventor: RENZI BRIVIO, Ludovico, I-37131 Verona (IT); LAVINI, Franco, I-37121 Verona (IT); FACCIOLI, Giovanni, I-46040 Monzambano (IT); VENTURINI, Daniele, I-37065 Povegliano Veronese (IT)
(74) Representative: Botti, Mario
(86) International application number: PCT/EP95/03305
(87) International publication number: WO 96/05777

(56) References cited:
- EP-A- 0 011 258
- EP-A- 0 517 939
- EP-A- 0 523 412
- EP-A- 0 546 460
- FR-A- 2 406 430
- FR-A- 2 688 399

## Description

### Field of application

This invention relates to an external trochanter splint, particularly for the stabilisation of pertrochanter and subtrochanter fractures by means of groups of bone screws inserted into the outer proximal third of the femur.

It is known that pertrochanter and persubtrochanter fractures are fractures of the proximal third of the femur which are by nature unstable and in which bone regrowth is difficult. These fractures are frequent in patients of medium to advanced age, who are normally subject to appreciable rarefaction of the metaphysis region due to osteoporosis, and may occur in victims of high energy impacts suffering from multiple injuries.

### State of the art

A known technique for stabilising trochanter fractures provides for the use of internal splint devices, i.e. those which are held within the limb until the fragments have become completely solid, such as endomedullary bolts, sheet-plate assemblies and screw-plate assemblies.

An alternative technique provides for the use of external stabilisation devices, i.e. those designed to be held partly outside, the limb over the patient's skin, which generally include two groups of bone screws which are located respectively in a proximal position close to the neck of the femur and in a distal position close to the knee, these being rigidly secured together by an external structure or frame.

Document EP 0 011 258 discloses an external splint for trochanteric fractures comprising two clamps coupled together by means of an intermediate member, so that the clamps are located apart from each other.

This invention relates to devices of the latter type.

One of the major disadvantages of external splints lies in the fact that the group of distal screws is so close to the knee that it limits its flexion, causing excessive stress on the tendon part of the tensor of the fascia lata with consequent accentuated pain in the muscle. This results in stiffening of the joint, which requires greater time and cost in order to recover mobility of the limb and rehabilitative physiotherapy.

A further disadvantage lies in the appreciable loss of blood associated with the implantation of the bone screws in the vicinity of the tensor muscle.

Another disadvantage lies in the frequent need for further surgical intervention to remove conventional splints, on account of their relative complexity.

### Brief description of the invention

The invention is defined by the features of independent claim 1.

The invention is designed to overcome the abovementioned disadvantages through a reliable, compact, comfortable and economic external trochanter splint.

A particular object is to provide an extremely compact trochanter splint which can be easily positioned by the surgeon.

Another object is to provide an external splint which eliminates interaction with the tendon part of the fascia lata permitting absolute mobility of the knee without any pain in the joint, in order to effect a drastic limitation in the time and costs of postoperative recovery.

A further object is to provide a trochanter splint which involves less vascularised areas and muscular tissues, in order to reduce blood loss during the operation.

A further object also consists of simplifying the device with the view to permitting the splint to be removed an out-patient basis without any complex operation in the operating theatre.

The invention accomplishes these objects through an external trochanter splint, particularly for the surgical stablilisation of femoral pertrochanter and subtrochanter fractures, comprising a pair of clamps having longitudinal axes respectively, one of the said clamps, the trochanter clamp, being adapted to releasably secure a first group of bone screws insertable into the mass of the trochanter and at least partly into the neck of the femur, the other clamp, the diaphysis clamp, being adapted to releasably secure a second group of bone screws insertable into the proximal diaphysis of the femur, an intermediate connecting member, first securing means to selectively immobilise the angle of divergence between the said longitudinal axes, and second immobilising means to selectively immobilise the angle of rotation of at least one of the said clamps about its own longitudinal axis, characterised in that said pair of clamps are coupled together relative to their longitudinal axes in a side-by-side position by means of said intermediate connecting member.

Preferably the intermediate member is connected to the clamps by means of a pair of rotatable joints having their own axes of rotation which are substantially at right angles.

Each joint may include a cylindrical seat on one side, and a pin of a complementary shape rotatably housed in the said seat on the other.

The body of the intermediate member has a central through hole which forms a seat for a first joint. It also has an external lateral appendage substantially perpendicular to the axis of the said central through hole defining a pin for a second joint.

In a particular embodiment one of the clamps has means to immobilise the trochanter screws with converging axes, and the other is subdivided into two parts which can be mutually orientated about the longitudinal axis of the clamp to offset the two groups of femoral screws at an angle.

In addition, the first joint has contact means comprising one or more surfaces of a frustoconical shape.

The external trochanter splint according to the invention provides appreciable stability for the fracture to be joined and increases the comfort of the patient, who can move freely immediately after the operation, reducing recovery times and hospital stays, with an appreciable benefit in economic terms.

In addition to this, the location of the splint in areas relatively far from the highly vascularised muscular tissues reduces blood losses.

The relative structural simplicity of the splint makes it possible to remove it on an out-patient basis avoiding a return to the operating theatre.

Finally, the possibility of orienting both the trochanter screws and the diaphysis screws of the respective clamps enormously increases the adaptability of the splint and its stability after installation in the case of complex or labile fractures.

### Brief description of the figures

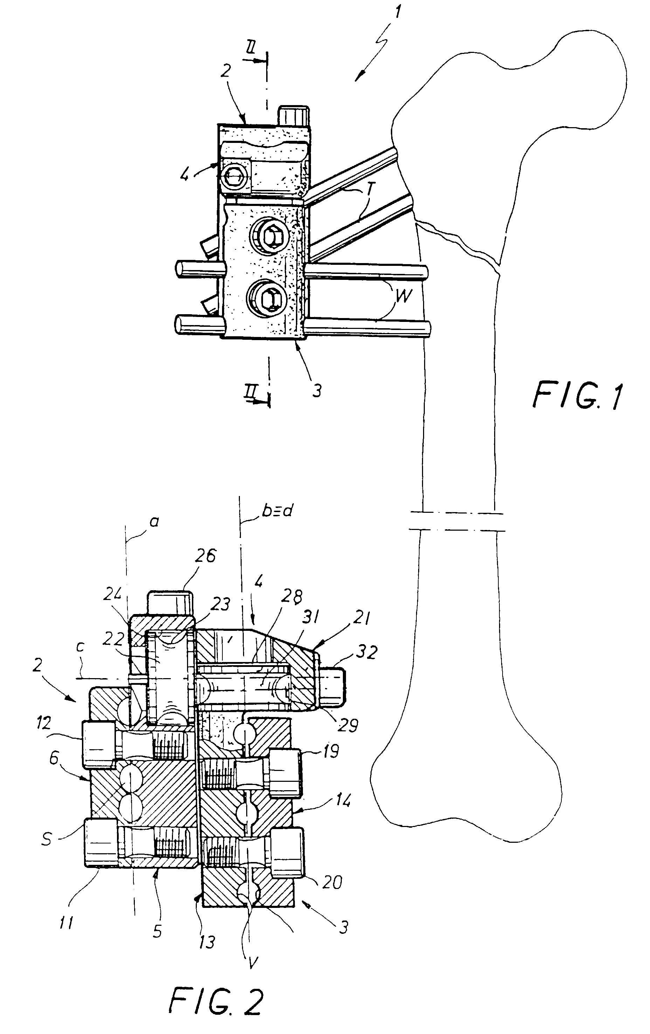

Further features and advantages of the invention will be more apparent from the description of a number of preferred, but not exclusive, embodiments of an external trochanter splint according to the invention illustrated as non limitative example and with reference to the accompanying drawings in which:-
Fig. 1 shows a lateral view of an external trochanter splint according to the invention applied to a subtrochanter femoral fracture;
Fig. 2 shows a front view of the external splint in Fig. 1 in partial cross-section along the plane of the line II-II;

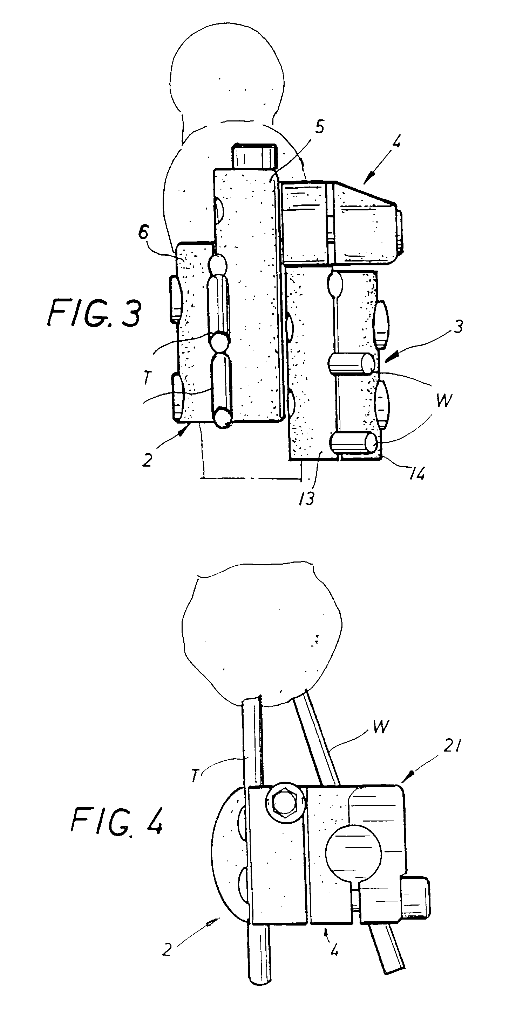

Fig. 3 shows a front view of the external splint in Fig. 1;
Fig. 4 shows a view from above of the external splint in Fig. 1;
Fig. 5 shows an exploded perspective view of the external splint in Fig. 1;
Fig. 6 shows a lateral view of an alternative embodiment of the external trochanter splint according to the invention applied to a relatively labile persubtrochanter fracture affecting the neck of the femure;
Fig. 7 shows a lateral view of the splint in Fig. 6 in partial cross-section in a plane along the line VII-VII;
Fig. 8 shows a front view of a detail of the external splint in Fig. 6;
Fig. 9 shows a view from above of the detail in Fig. 8;
Fig. 10 shows a front view of the external splint in Fig. 6;
Fig. 11 shows a view from above of the external splint in Fig. 6; and
Fig. 12 shows an exploded perspective view of the external splint in Fig. 6.

### Description of a preferred embodiment

With reference to the Figs. mentioned, a trochanter splint according to the invention, indicated as a whole by reference number 1, comprises a pair of clamps 2, 3 connected by an intermediate member 4.

Each clamp comprises two superimposable members, a base and a cover respectively, which can be coupled together by suitable tightening means.

In particular, first clamp 2 comprises a base 5 and a cover 6 which have respective internal faces which are substantially flat. In the illustrated embodiment, base 5 is of a prismatic shape with an external face which is also flat, while the cover has a rounded external face to avoid edges and to harmonise with the limb. The two members 5, 6 have pairs of aligned through holes, respectively a pair of smooth holes 7, 8 in the cover and a pair of threaded holes 9, 10 in the base, for recessed-head locking bolts 11, 12.

Once joined together the two members 5, 6 define a longitudinal axis a for clamp 2 resulting from the intersection of the plane separating the flat faces of the members with a plane perpendicular to the internal faces passing through the middle thereof.

On the internal faces of the two members 5, 6 a first set of transverse seats R are provided substantially parallel and in opposing positions. Seats R are inclined with respect to axis a at an angle a approximately equal to that of the mean inclination of the neck of the trochanter with respect to the axis of the femur. Provision may be made in each member 5, 6 for a second set of seats S which are symmetrical with the first with reference to the mid line of the internal face in order to permit fitting to either a right limb or a left limb.

By tightening bolts 11, 12 and members 5, 6 it is possible to stably immobilise a set of bone screws V, previously located in the mass of the trochanter, so that they pass partly through the neck and the head of the femur. For this reason clamp 2 is referred to hereinafter merely as the trochanter clamp. It will be noted that once tightened a very small space remains between the two members and this reduces the problem of the differentiated immobilisation of the bolts which occurs when one bolt is tightened down before another.

Similarly, second clamp 3 comprises a base 13 and a cover 14, both having substantially flat internal faces and rounded external faces so as generally to have the shape of half cylinders. In this case again members 13, 14 have through holes, smooth holes 15, 16 in cover 14 and threaded holes 17, 18 in base 13 respectively, to house recessed-head locking bolts 19, 20. Once joined together, the clamp has a longitudinal axis b defined as previously indicated for clamp 2.

On the internal faces of members 13, 14 there is provided a set of transverse seats V substantially parallel and in opposing positions. In this case seats V are substantially perpendicular to longitudinal axis b in that they are designed to stably immobilise a series of bone screws W previously inserted in the proximal diaphysis of the femur. For this reason clamp 3 is referred to hereinafter as the diaphysis clamp. It will be noted that these screws W have the advantage of being located in a position sufficiently distant from the tendon part of the fascia lata so as to avoid pain and limiting movement of the knee joint, which is a typical feature of conventional external trochanter splints.

Intermediate member 4 couples together the two clamps 2, 3 in side-by-side positions selectively determining their relative orientation by means of their respective rotating joints.

In particular, connecting member 4 has a body 21 of an approximately prismatic shape defining two axes at right angles, c and d respectively. An extension 22 of approximately cylindrical shape having an axis c and a peripheral groove 23 is formed on body 21. Extension 22 defines a pin which is designed to engage in a corresponding seat 24 provided in an accurate free manner in base 5 so as to define a first rotating coupling joint. Seat 24 yields elastically in that base 5 has a radial notch 25 in the region of minimum thickness. A securing bolt which can be inserted into a partly threaded hole 27 located in base 5 is provided. Appropriately, inserting bolt 26 in hole 27 after extension 22 of intermediate member 4 has been housed in seat 24 avoids involuntary detachment of trochanter clamp 2 from intermediate member 4. Tightening down bolts 26 immobilises the relative angle of base 5, and therefore clamp 2, with respect to the axis of d of intermediate member 4.

The second joint is defined by an axial extension 28 of base 13 of clamp 3, of a substantially cylindrical shape, which can be inserted into an internal seat 29 of a free and accurate corresponding shape formed in body 21 of member 4 having axis d. Seat 29 can yield elastically owing to the presence of a radial slit 30 on its lateral wall, preferably in the area of minimum thickness. Again in this case axial extension 28, which defines the pin of the second joint, has a peripheral groove 31 for the passage of a bolt 32 inserted into a hole 33 which is partially threaded internally and provided in body 21 of member 4 which serves to enclose seat 29 elastically. When bolt 32 is inserted into hole 33 this secures axial extension 28 avoiding separation of diaphysis clamp 3 from intermediate member 4.

In use, the surgeon inserts at least one pair of screws V into the mass of the trochanter and at least one pair of screws W into the proximal diaphysis zone of the femur, in suitable holes previously made in the bone through the use of a guide mask, or the said clamps used as drilling guides.

After this, bolts 26 and 32 are slackened off releasing the rotating joints which connect intermediate connecting member 4 to clamps 2 and 3. Clamps 2, 3 are then orientated, varying the angle of divergence between their respective axes a, b and the clamp 3 around its own axis b. Bolts 11, 12, 19, 20 are then tightened, immobilising clamp members 2, 3 to their corresponding groups of bone screws V, W. At this point, the surgeon, using a brilliance emitter, can set the fracture in two or one or more exposure planes. Finally, bolts 26, 32 are tightened, stabilising the fracture.

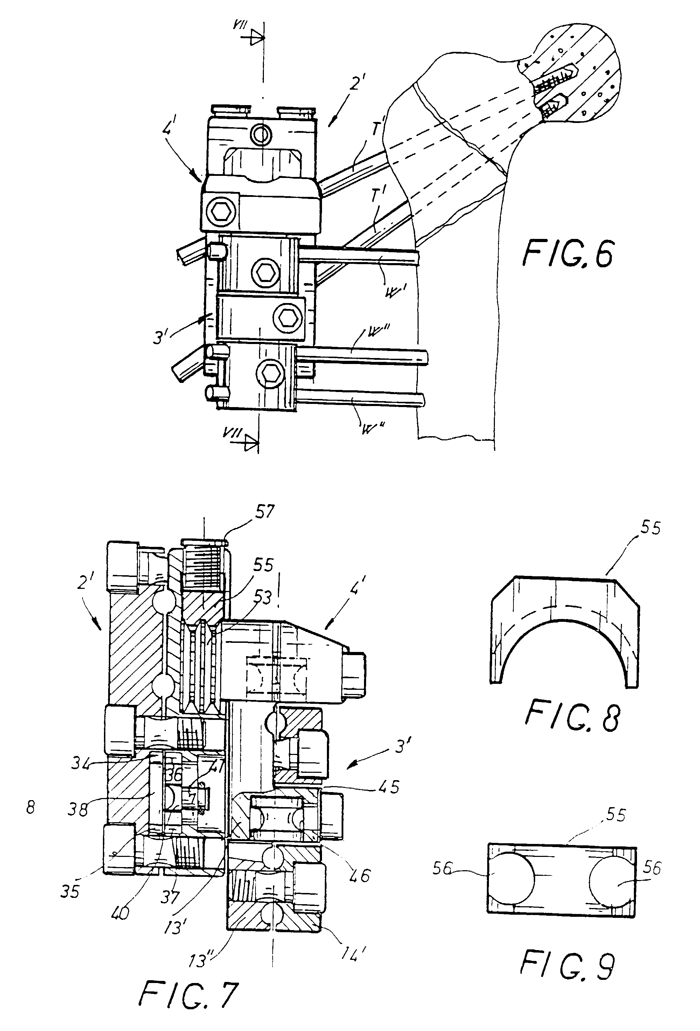

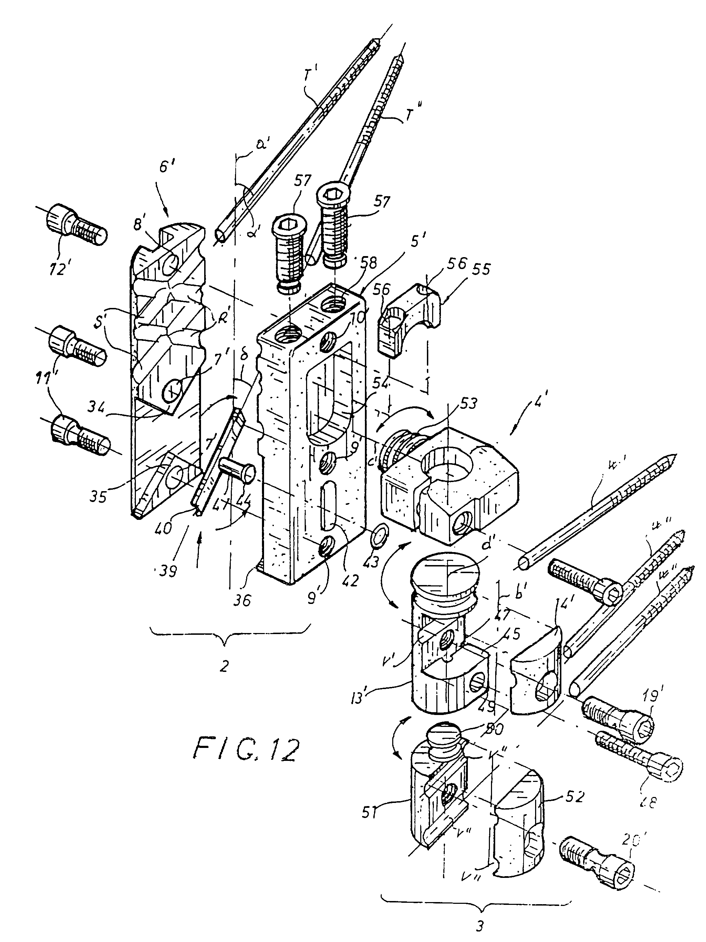

The alternative embodiment illustrated in Figs. 6-12 differs from that in the preceding figures in three special technical features affecting the two clamps and a joint between them. These differences are described below, using reference numbers with apostrophes where necessary to identify the main corresponding details.

The first difference relates to the structure of trochanter clamp 2' and in particular its means for securing the trochanter screws. As in the previous embodiment, the clamp comprises a base 5' and a cover 6' which face each other and are secured by securing bolts 11', 12' with recessed hexagonal heads which pass through corresponding smooth holes 7', 8' provided in cover 6' and engage threaded holes 9', 10' provided in base 5'.

A first set of parallel fixed transverse seats R', inclined at an angle α with respect to the axis of the clamp a' to immobilise one or two bone screws which pass through the mass of the trochanter and part of the neck of the femur, are provided on the upper part of the flat face of base 5' and cover 6' in facing positions. Again in this case, in addition to the first set of seats R' there is a second set S' which is symmetrical with respect to the above so that the clamp can be used on either the left or right limb. It will be noted that these means for securing the trochanter screws are of the fixed type and hold them parallel.

It has been verified clinically that the screws which pass through the neck of the femur can slip if they are perfectly parallel, i.e. they can progressively penetrate the bone tissue as a result of the mechanical stresses acting on the femur, to the extent that their ends can even project from the head of the femur, immobilising the joint.

One means of avoiding this disadvantage is to provide non-parallel screws i.e. slightly converging screws, while keeping them in the same vertical plane. However, it is not easy to provide a precise suitable angle of convergence for every configuration and every break in the femur.

For this reason it was decided to add an adjustable securing point to the upper fixed securing points. With this object, the lower part of the inside flat face of cover 6' has a butterfly-shaped slot or recess leaving two projections 34, 35 with V-shaped sides corresponding to holes 7'. On the opposite side of base 5' there is an identical recess which defines corresponding projections 36, 37 which are a mirror image of the above. When the two members are coupled together, the said recesses form a cavity 38 which is capable of housing a support 39 for a trochanter screw. The support comprises a channel member 40 from which extends a perpendicular pin 41 which can be inserted into an elongated hole 42 in base 5' with freedom to rotate and slide longitudinally. After pin 41 has been inserted in hole 42, an O-ring 43 is fitted on the end of the pin, and is retained by the enlarged head (44) of pin 41 to prevent the pin from being drawn out in an axial direction. when bolts 11', 12' are tightened, screw T" is securely immobilised between support 39 and the opposite face of cover 6'. As a result of this arrangement the surgeon can vary the angle of inclination δ formed between the lower trochanter screw T" and the axis of the clamp at will, so as to make this screw converge towards the other upper trochanter screw or screws T', avoiding the danger of breaking the head.

The second modification relates to the diaphysis clamp, in particular its method of securing the corresponding bone screws. From the practical point of view some difficulties have been encountered with the insertion of these screws, especially the one furthest from the head of the femur, difficulties which largely depend on the accuracy with which the screws inserted into the neck of the femur are fitted.

In order to ease this operation it was decided to modify the diaphysis clamp so as to allow the distal diaphysis screws to be orientated with respect to the proximal screws, in practice using a third lockable joint with an axis identical to that of the axis d' of clamp 3' itself.

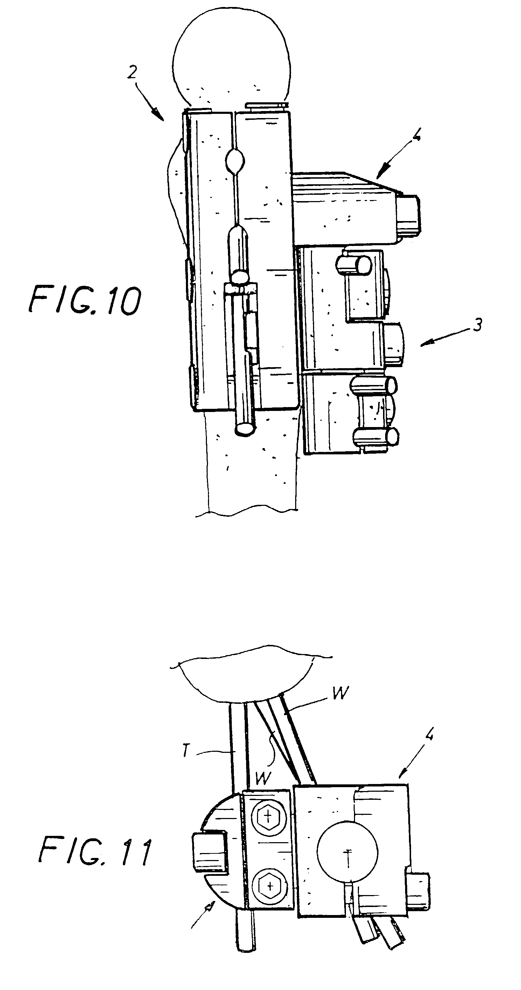

To this aim diaphysis clamp 3' comprises an upper base 13', generally in the shape of a half cylinder, and an upper cover 14' tightened by a bolt 19' immobilising a first bone screw W' in opposing seats V'. The lower part of base 13' has an expansion 45 with an internal cylindrical cavity 46 which is coaxial with the clamp, and opens downwards, as may be seen in Fig. 7, and is provided with a radial notch 47 to make it elastically closable. A bolt 48 is inserted into a hole 49 of expansion 45 to secure cavity 46. Cavity 46 houses an axial expansion 50 of a lower base 51, also in the form of a half cylinder, with minimum play but with the ability to rotate about the clamp axis b', and which can also be immobilised in a preselected orientation by tightening bolt 48. The assembly comprising axial expansion 50 inserted into cavity 46 which can be immobilised by bolt 48 forms the third rotating joint of the splint. A lower cover 52 is coupled to lower base 51 by means of a bolt 20' to immobilise a second and possibly a third diaphysis screw W" in opposing seats V".

In this way the surgeon is able to insert the lower diaphysis screw into the hole provided by conventional means without any difficulty, without depending on the accuracy with which the screws are positioned in the neck of the femur. Also if the femoral screws have to be repositioned and their corresponding holes have to be remade, this can easily be performed in a position which is rotated with respect to the previous position, guaranteeing stability of the setting.

The third modification relates to the nature of the quick-lock joints placed between intermediate member 4 and clamps 2, 3. In the embodiment illustrated in Figs. 6-12, for simplicity only one of the joints has been subjected to this modification, in particular the one between member 4' and clamp 2' and axis c.

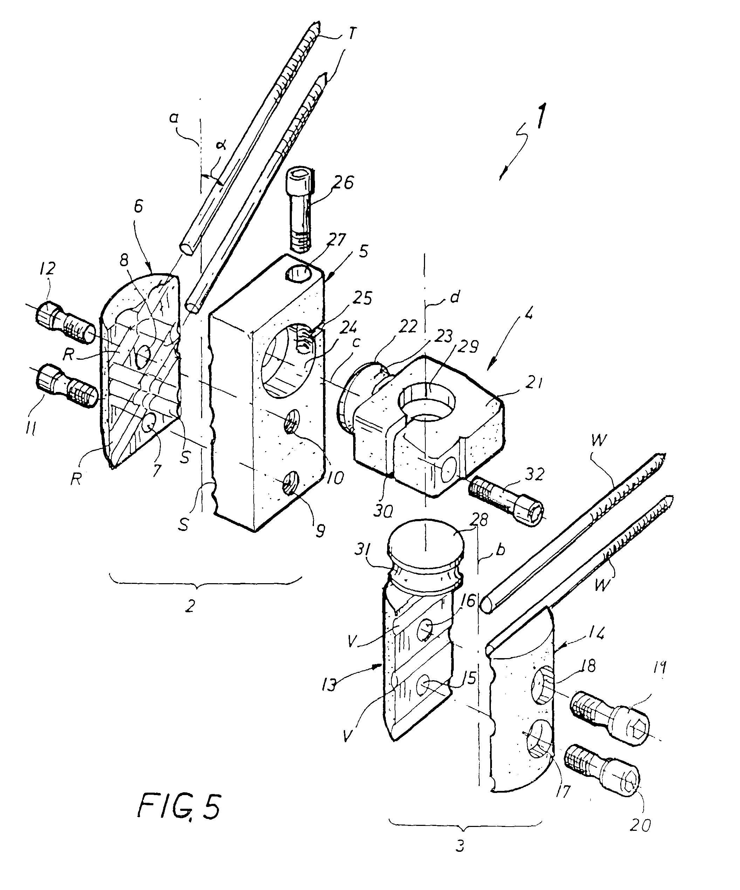

Instead of the substantially cylindrical axial extension 22 with a groove 23 of semicircular cross-section for passage of securing bolt 32, as illustrated in Fig. 5, a substantially cylindrical appendix 53 with multiple grooves having a radial cross-section in the form of a trapezium or wedge, which can clearly be seen in Fig. 7, is provided. Appendix 53 is housed in the semicircular base of cavity 54 in base 5' and is held in position by immobilising rod 55, having a contact surface which is approximately semicircular and in a complementary shape to that of appendix 53 itself, i.e. with trapezoidal grooves. On the upper part of rod 55 there are two cylindrical seats 56 against which the flat ends of a pair of pressure bolts 57 act, and these in turn are threaded into corresponding threaded holes 58 formed on the upper edge of base 5'.

When bolts 57 are tightened, restraining rod 55 is pressed against appendix 53. The contact surfaces of complementary shape exert a mutual wedging effect which increases the resistant torque without excessively stressing the main structure of base 5' and increases the security of the joint. It is obvious that this type of locking structure may also be applied in general to the other joint with similar benefits in terms of security and strength.

The materials used for the members comprising clamps 2, 3, 2', 3' and for intermediate connecting member 4, 4' may be selected from those having a high strength/weight ratio which are biologically compatible and can be sterilised in an autoclave, such as high strength aluminium alloys (UN19007/2, ERGALL 55) with hard black oxidised surface treatment. Thee bolts may be constructed from high strength stainless steel of the type AISI 303 or AISI 304, which have been tumbled, passivated and electropolished.

It will be noted that one of covers 5, 5', 8, 8' or both may be joined by means of slides or elastic attachments to offer a minimum amount of longitudinal shift, in order to make the splint dynamic and encourage regeneration of the bony tissue at the focus of the fracture.

## Claims

1. An external trochanter splint, particularly for the surgical stabilisation of femoral pertrochanter and subtrochanter fractures, comprising a pair of clamps (2,3) having longitudinal axes (a, b) respectively, one of the said clamps, the trochanter clamp (2), being adapted to releasably secure a first group of bone screws (V), insertable into the mass of the trochanter and at least partly into the neck of the femur, the other clamp, the diaphysis clamp (3), being adapted to releasably secure a second group of bone screws (W) insertable into the proximal diaphysis of the femur, an intermediate connecting member (4) first securing means (26) to selectively immobilise the angle of divergence between the said longitudinal axes (a, b), and second immobilising means (32) to selectively immobilise the angle of rotation of at least one of the said clamps (3) about its own longitudinal axis (b), characterised in that said pair of clamps (2,3) are coupled together relative to their longitudinal axes (a, b) in a side-by-side position by means of said intermediate connecting member (4).

2. An external splint according to claim 1, in which the said intermediate member (4) comprises a central body (21) connected to the said clamps by a pair of joints which are rotatable about their respective rotation axes (c, d), which axes are substantially at right angles.

3. An external splint according to claim 2, in which each joint comprises a cylindrical seat on the one side and an extension of complementary shape rotatably housed in the said seat on the other side.

4. An external splint according to claim 3, in which the said body (21) having an internal cavity (29) defining a seat for a first rotating joint.

5. An external splint according to claim 4, in which the said body (21) has an external lateral extension (22) having an axis (c) which is substantially perpendicular to the axis (d) of the said seat (29) defining a pin for a second joint.

6. An external splint according to claim 3, in which each of the said clamps (2,3) comprises a base (6,7) and a cover (5,8) having substantially flat respective internal faces, the said base and the said cover being coupled together by means of at least one securing bolt (11, 12; 19, 20).

7. An external splint according to claim 6, in which the internal faces of the base and the cover in each clamp have at least one set of seats (R, V) to house the said groups of bone screws (T, W).

8. An external splint according to claim 7, in which the seats (V) housing the screws of the diaphysis clamp (3) are substantially perpendicular to the axis (b) of the said clamp.

9. An external splint according to claim 6, in which the seats (R) housing the screws of the said trochanter clamp (2) are inclined with respect to the longitudinal axis (a) by a divergence angle (α) which is approximately equal to the average inclination angle of the neck of the trochanter with respect to that of the femur.

10. An external splint according to claim 9, in which the said trochanter clamp (2) has a second set of inclined seats (S), symmetrical to those of the first set, (R) with respect to longitudinal axis (a) on the internal faces of base (5) and cover (6) in order to permit fitting either to a right or left limb.

11. An external splint according to claim 6, in which the base (13) of one of the said clamps (3) has an axial extension (28) defining a pin for the said first joint towards one proximal end, the said pin being capable of being inserted with a free and snug fitting into the said seat (29) formed in the body (21) of the said intermediate member (4).

12. An external splint according to claim 11, in which the base (6) of the other of the said clamps (2) has a transverse through hole (24) towards one of its longitudinal ends which defines a seat for the said second joint, and which seat being adapted to house the said cylindrical extension (22) of the said intermediate body with a free and snug fitting.

13. An external splint according to claim 12, in which the base (6) of the clamp provided with the said transverse hold (24) has a substantially diametral notch (25) with respect to the said hold (24) in order to make the seat of the said second joint elastically yielding.

14. An external splint according to claim 13, in which the said first locking means comprise a first bolt (26) tangential to the said transverse hole so as to close off selectively the seat (24) of the said second joint after the said lateral appendage (22) of the said intermediate member (4) has been orientated at will in order to adjust the divergence angle (α) between the said clamps.

15. An external splint according to claim 14, in which the body (21) of the said intermediate member (4) has a slit (30) which is substantially diametral to the said central hole and is capable of making the seat of the said first joint open and elastically yielding.

16. An external splint according to claim 3, in which the said second immobilising means comprise a second immobilising bolt (32), tangential to the said seat (29) of the said body (21), capable of closing it off elastically to vary the angle of rotation of one of the said clamps about its own longitudinal axis (d).

17. An external splint according to claim 9, in which at least one seat for a bone screw in the trochanter clamp (2') can be orientated in a plane parallel to the surface separating the base (5') and the cover (6').

18. An external splint according to claim 17, in which the said orientatable seat comprises a support (39) with a channel member (40) adapted to house a bone screw (T") from which extends a pin (41) which is inserted with freedom to rotate and slide in an elongated hole (42) formed in the said base (5') so as to vary the angle of inclination (6) with respect to the axis (a') of the clamp (2').

19. An external splint according to claim 9, in which the said diaphysis clamp comprises an upper base (13') connected to the said intermediate member (4') by said first joint, and a lower base (51) joined to the said upper base by means of a third rotating joint with an axis (b') which is coincident with that of clamp (3').

20. An external splint according to claim 19, in which the said third joint comprises an axial extension (50) formed on the said lower base (51) and inserted into a cylindrical cavity (46) formed in an expansion (45) of the said upper base (13'), which can be immobilised in position by means of a locking bolt (48) screwed onto the said expansion (45).

21. An external splint according to claim 11, in which the base (6') of the said trochanter clamp (2') has a cavity (54) forming a seat for the said second joint, towards its longitudinal end, this seat being capable of housing an appendix (53) of the intermediate member (4') with a free and snug fitting in which the external surface of the said appendix (53) is substantially cylindrical with one or more grooves having a substantially trapezoidal radial cross-section.

22. An external splint according to claim 21, in which a locking rod (55) is mounted between the said appendix (53) and the said cavity (54), and is secured against the said appendage by means of one or more pressure bolts (57) screwed onto the said base (5'), the said rod having an internal semi-cylindrical surface which compliments that of the said appendix (53) in order to exert a wedging effect on the said appendix.

## Patentansprüche

1. Externe Trochanterschiene insbesondere zur chirurgischen Stabilisierung von femoralen Pertrochanter- und Subtrochanterfrakturen mit einem Klemmenpaar (2,3) mit Längsachsen (a, b), wobei eine der Klemmen (2), d.h. die Trochanterklemme, zur lösbaren Befestigung einer ersten Gruppe von Knochenschrauben (v) vorgesehen ist, die in die Trochantermasse und zumindest teilweise in den Oberschenkelhals eingeführt werden können, und die andere Klemme (3), d.h. die Diaphyseklemme, zur lösbaren Befestigung einer zweiten Gruppe von Knochenschrauben (W) dient, welche in die proximale Diaphyse des Oberschenkels eingeführt werden können, ein Zwischenverbindungsglied (4), wobei erste Befestigungsvorrichtungen (26) vorgesehen sind, um den Divergenzwinkel zwischen den Längsachsen (a, b) selektiv einzustellen, und zweite Feststellvorrichtungen (32) dazu dienen, um den Drehwinkel mindestens einer der Klemmen (3) um ihre eigene Längsachse (b) selektiv einzustellen, dadurch gekennzeichnet, daß das Klemmenpaar (2,3) relativ zu seinen Längsachsen (b, c) nebeneinanderliegend durch besagtes Zwischenverbindungsglied miteinander verbunden wird.

2. Externe Schiene nach Anspruch 1, dadurch gekennzeichnet, dass das Zwischenverbindungselement (4) ein Mittelteil (21) umfasst, das mit den Klemmen über ein Paar Gelenke verbunden ist, welche um die jeweiligen Drehachsen (c, d) drehbar sind, wobei die Achsen weitgehend im rechten Winkel verlaufen.

3. Externe Schiene nach Anspruch 2, dadurch gekennzeichnet, dass jedes Gelenk auf der einen Seite einen zylindrischen Sitz aufweist und auf der anderen Seite eine Verlängerung von entsprechender Form besitzt, welche im Sitz drehbar gelagert ist.

4. Externe Schiene nach Anspruch 3, dadurch gekennzeichnet, dass das Mittelteil (21) eine innere Ausnehmung (29) besitzt, die einen Sitz für ein erstes Drehgelenk bildet.

5. Externe Schiene nach Anspruch 4, dadurch gekennzeichnet, dass das Mittelteil (21) eine externe seitliche Verlängerung (22) mit einer Achse (c) aufweist, die weitgehend senkrecht zur Achse (d) des Sitzes (29) verläuft und eine Stift für ein zweites Gelenk bildet.

6. Externe Schiene nach Anspruch 3, dadurch gekennzeichnet, dass jede der Klemmen (2,3) ein Unterteil (6,7) und eine Abdeckung (5,8) mit weitgehend flachen Innenflächen aufweist, wobei das Unterteil und die Abdeckung durch mindestens eine Befestigungsschraube (11,12; 19,20) miteinander verbunden sind.

7. Externe Schiene nach Anspruch 6, dadurch gekennzeichnet, dass die Innenflächen des Unterteils und der Abdeckung einer jeden Klemme mindestens einen Satz sitze (R, V) aufweisen, um die jeweiligen Gruppen von Knochenschrauben (T, W) aufzunehmen.

8. Externe Schiene nach Anspruch 7, dadurch gekennzeichnet, dass die Sitze (V) zur Aufnahme der Schrauben der Diaphyseklemme (3) weitgehend senkrecht zur Achse (b) der Klemme verlaufen.

9. Externe Schiene nach Anspruch 6, dadurch gekennzeichnet, dass die Sitze (R) zur Aufnahme der Schrauben der Trochanterklemme (2) relativ zur Längsachse (a) unter einem Divergenzwinkel (α) geneigt sind, der in etwa gleich dem durchschnittlichen Neigungswinkel des Trochanterhalses relativ zu dem des Oberschenkels ist.

10. Externe Schiene nach Anspruch 9, dadurch gekennzeichnet, dass die Trochanterklemme (2) einen zweiten Satz geneigter Sitze (S) umfasst, die symmetrisch zu denen des ersten Satzes (R) relativ zur Längsachse (a) auf den Innenflächen des Unterteils (5) und der Abdeckung (6) angeordnet sind, um ein Anlegen. an einer rechten oder einer linken Extremität zu ermöglichen.

11. Externe Schiene nach Anspruch 6, dadurch gekennzeichnet, dass das Unteteil (13) einer der Klemmen (3) eine axiale Verlängerung (28) aufweist, die einen Stift für das erste Gelenk in Richtung eines proximalen Endes bildet, wobei der Stift freigängig und mit enger Passung in den im Mittelteil (21) des Zwischenverbindungsglieds (4) ausgebildeten Sitz (29) eingeführt werden kann.

12. Externe Schiene nach Anspruch 11, dadurch gekennzeichnet, dass das Unterteil (6) der anderen Klemme (2) eine in Querrichtung verlaufende Durchgangsöffnung (24) in Richtung eines ihrer Längsenden besitzt, die einen Sitz für das zweite Gelenk bildet, wobei dieser Sitz zur Aufnahme der zylindrischen Verlängerung (22) des Zwischenglieds mit freigängiger und enger Passung dient.

13. Externe Schiene nach Anspruch 12, dadurch gekennzeichnet, dass das Unterteil (6) der mit der in Querrichtung verlaufenden Halterung (24) versehenen Klemme eine weitgehend diametrale Einkerbung (25) relativ zur Halterung (24) aufweist, damit der Sitz für das zweite Gelenk elastisch nachgeben kann.

14. Externe Schiene nach Anspruch 13, dadurch gekennzeichnet, dass die ersten Befestigungsvorrichtungen eine erste Schraube (26) tangential zu der in Querrichtung verlaufenden Öffnung umfassen, um den Sitz (24) des zweiten Gelenks selektiv zu verschliessen, nachdem der seitliche Anhang (22) des Zwischenglieds (4) beliebig ausgerichtet worden ist, um den Divergenzwinkel (α) zwischen den Klemmen einzustellen.

15. Externe Schiene nach Anspruch 14, dadurch gekennzeichnet, dass das Mittelteil (21) des Zwischenglieds (4) einen Schlitz (30) aufweist, der im wesentlichen diametral zur mittleren Öffnung verläuft und die Möglichkeit bietet, den Sitz des ersten Gelenks zu öffnen und elastisch nachgebend zu gestalten.

16. Externe Schiene nach Anspruch 3, dadurch gekennzeichnet, dass die zweiten Befestigungsvorrichtungen eine zweite Feststellschraube (32) tangential zum Sitz (29) des Mittelteils (21) umfassen, die in der Lage ist, den Sitz elastisch zu verschliessen, um den Drehwinkel einer der Klemmen um ihre eigene Längsachse (d) zu verändern.

17. Externe Schiene nach Anspruch 9, dadurch gekennzeichnet, dass mindestens ein Sitz für eine Knochenschraube in der Trochanterklemme (2') in einer Ebene parallel zu der Oberfläche verstellt werden kann, welche das Unterteil (5') und die Abdeckung (6') voneinander trennt.

18. Externe Schiene nach Anspruch 17, dadurch gekennzeichnet, dass der verstellbare Sitz eine Halterung (39) mit einem Kanalelement (40) zur Aufnahme einer Knochenschraube (T") umfasst, von dem ein Stift (41) ausgeht, der frei drehbar und verschiebbar in ein Langloch (42) im Unterteil (5') eingesetzt werden kann, um den Neigungswinkel (δ) relativ zur Achse (a') der Klemme (2') Zu verändern.

19. Externe Schiene nach Anspruch 9, dadurch gekennzeichnet, dass die Diaphyseklemme ein oberes Unterteil (13') aufweist, das mit dem Zwischenglied (4') über das erste Gelenk verbunden ist, und ein unteres Unterteil (51) umfasst, das mit dem oberen unterteil über ein drittes Drehgelenk mit einer Achse (b') in Verbindung steht, die mit der Achse der Klemme (3') zusammenfällt.

20. Externe Schiene nach Anspruch 19, dadurch gekennzeichnet, dass das dritte Gelenk eine axiale Verlängerung (50) aufweist, die im unteren Unterteil (51) ausgebildet ist und in eine zylindrische Ausnehmung (46) in einer Erweiterung (45) des oberen Unterteils (13') eingeführt wird, dessen Position durch eine auf die Erweiterung (45) aufgeschraubte Feststellschraube (48) gesichert werden kann.

21. Externe Schiene nach Anspruch 11, dadurch gekennzeichnet, dass das Unterteil (6') der Trochanterklemme (2') eine Ausnehmung (54) aufweist, die zum Längsende hin einen Sitz für das zweite Gelenk bildet, wobei der Sitz in der Lage ist, einen Anhang (53) des Zwischenglieds (4') mit einer freigangigen und engen Passung aufzunehmen, und wobei die Aussenfläche des Anhangs (53) weitgehend zylindrisch ausgebildet und mit einer oder mehreren Nuten versehen ist, die einen im wesentlichen trapezförmigen radialen Querschnitt aufweist/aufweisen.

22. Externe Schiene nach Anspruch 21, dadurch gekennzeichnet, dass eine Verriegelungsstange (55) zwischen dem Anhang (53) und der Ausnehmung (54) angeordnet und gegen den Anhang mittels einer oder mehrerer Druckschrauben (57) befestigt ist, die auf das Unterteil (5') aufgeschraubt ist/sind, wobei die Stange eine halbzylindrische Innenfläche aufweist, welche der des Anhangs (53) angepasst ist, um eine Keilwirkung auf den Anhang auszuüben.

## Revendications

1. Attèle externe destinée au trochanter, particulièrement pour la stabilisation chirurgicale de fractures du pertrochanter et du subtrochanter fémoral, comportant une paire de pinces (2, 3) ayant des axes longitudinaux (a, b) respectifs, une desdites pinces, la pince de trochanter (2), étant conçue pour fixer de façon amovible un premier groupe de vis d'os (V) susceptibles d'être insérées dans la masse du trochanter et au moins partiellement dans le col du fémur, l'autre pince, la pince de diaphyse (3), étant conçue pour fixer de façon amovible un deuxième groupe de vis d'os (W) susceptibles d'être insérées dans la diaphyse proximale du fémur, un organe de connexion intermédiaire (4), des premiers moyens de fixation (26) pour immobiliser sélectivement l'angle de divergence entre lesdits axes longitudinaux (a, b) et des seconds moyens d'immobilisation (32) pour immobiliser sélectivement l'angle de rotation d'au moins une desdites pinces (3) autour de son propre axe longitudinal (b), *caractérisée en ce que* lesdites paires de pinces (2, 3) sont accouplées ensemble en position côte à côte par rapport à leurs axes longitudinaux (a, b) par l'intermédiaire dudit organe de connexion intermédiaire.

2. Attèle externe selon la revendication 1, caractérisée en ce que ledit organe intermédiaire (4) comporte un corps central (21) connecté auxdites pinces par une paire de joints montés rotatifs autour de leurs axes de rotation respectifs (c, d), ces axes étant sensiblement perpendiculaires.

3. Attèle externe selon la revendication 2, caractérisée en ce que chaque joint comporte d'un côté un siège cylindrique et de l'autre une extension de forme complémentaire logée, de façon à pouvoir tourner, dans ledit siège.

4. Attèle externe selon la revendication 3, caractérisée en ce que ledit corps (21) possède une cavité interne (29) définissant un siège pour le premier joint rotatif.

5. Attèle externe selon la revendication 4, caractérisée en ce que ledit corps (21) possède une extension latérale externe (22) ayant un axe (c) sensiblement perpendiculaire à l'axe (d) dudit siège (29) définissant une broche pour un deuxième joint.

6. Attèle externe selon la revendication 3, caractérisée en ce que chacune desdites pinces (2, 3) comporte une base (6, 7) et un couvercle (5, 8) ayant des faces internes respectives sensiblement plates, ladite base et ledit couvercle étant couplés ensemble au moyen d'au moins un boulon de fixation (11, 12 ; 19, 20).

7. Attèle externe selon la revendication 6, caractérisée en ce que les faces internes de la base et du couvercle de chaque pince comportent au moins un jeu de sièges (R, V) pour loger lesdits groupes de vis d'os (T, W).

8. Attèle externe selon la revendication 7, caractérisée en ce que les sièges (V), logeant les vis de la pince de diaphyse (3), sont sensiblement perpendiculaires à l'axe (b) de ladite pince.

9. Attèle externe selon la revendication 6, caractérisée en ce que les sièges (R), logeant les vis de ladite pince de trochanter (2), sont inclinés par rapport à l'axe longitudinal (a), d'un angle de divergence (α) approximativement égal à l'angle d'inclinaison moyen du col du trochanter par rapport à celui du fémur.

10. Attèle externe selon la revendication 9, caractérisée en ce que ladite pince de trochanter (2) possède un deuxième jeu de sièges inclinés (S), symétriques à ceux du premier jeu (R) par rapport à l'axe longitudinal (a) sur les faces internes de la base (5) et du couvercle (6), afin de permettre de s'adapter à un membre droit ou gauche.

11. Attèle externe selon la revendication 6, caractérisée en ce que la base (13) d'une desdites pinces (3) possède une extension axiale (28) définissant une broche pour ledit premier joint dirigé vers l'extrémité proximale, ladite broche pouvant s'insérer avec un emboîtement libre et ajusté dans ledit siège (29) formé dans le corps (21) dudit organe intermédiaire (4).

12. Attèle externe selon la revendication 11, caractérisée en ce que la base (6) de l'autre desdites pinces (2) possède un trou traversant transversal (24) dirigé vers une de ses extrémités définissant un siège pour ledit deuxième joint, ledit siège étant conçu pour loger ladite extension cylindrique (22) dudit corps intermédiaire avec un emboîtement libre et ajusté.

13. Attèle externe selon la revendication 12, caractérisée en ce que la base (6) de la pince munie dudit trou transversal (24) possède une encoche (25) dirigée sensiblement diamétralement par rapport audit trou (24) afin de rendre flexible et élastique le siège de ladite deuxième jointure.

14. Attèle externe selon la revendication 13, caractérisée en ce que lesdits premiers moyens de verrouillage comportent un premier boulon (26) tangentiel audit trou transversal, de façon à refermer sélectivement le siège (24) dudit deuxième joint, après que ladite extension latérale (22) dudit organe intermédiaire (4) ait été orientée à volonté pour régler l'angle de divergence (α) entre lesdites pinces.

15. Attèle externe selon la revendication 14, caractérisée en ce que le corps (21) dudit organe intermédiaire (4) possède une fente (30) dirigée sensiblement diamétralement par rapport audit trou central et conçue pour ouvrir et rendre flexible et élastique le siège de ladite première jointure.

16. Attèle externe selon la revendication 3, caractérisée en ce que lesdits deuxièmes moyens d'immobilisation comportent un deuxième boulon d'immobilisation (32), tangentiel audit siège (29) dudit corps (21), conçu pour le refermer élastiquement afin de faire varier l'angle de rotation d'une desdites pinces autour de son propre axe longitudinal (d).

17. Attèle externe selon la revendication 9, caractérisée en ce qu'au moins un siège de vis d'os dans la pince de trochanter (2') peut être orienté dans un plan parallèle à la surface séparant la base (5') du couvercle (6').

18. Attèle externe selon la revendication 17, caractérisée en ce que ledit siège orientable comporte un support (39) muni d'un canal (40) conçu pour loger une vis d'os (T") à partir duquel s'étend une broche (41) insérée, avec la faculté de tourner et de glisser, dans un trou allongé (42) formé dans ladite base (5'), de façon à faire varier l'angle d'inclinaison (δ) par rapport à l'axe (a') de la pince (2').

19. Attèle externe selon la revendication 9, caractérisée en ce que ladite pince de diaphyse comporte une base supérieure (13') connectée audit organe intermédiaire (4') par ladite première jointure et une base inférieure reliée à ladite base supérieure au moyen d'une troisième jointure rotative, avec un axe (b') qui coïncide avec celui de la pince (3').

20. Attèle externe selon la revendication 19, caractérisée en ce que ledit troisième joint comporte une extension axiale (50) formée sur ladite base inférieure (51) et insérée dans une cavité cylindrique (46) formée dans une extension (45) de ladite base supérieure (13'), celle-ci pouvant être immobilisée en place au moyen d'un boulon de verrouillage (48) vissé sur ladite extension (45).

21. Attèle externe selon la revendication 11, caractérisée en ce que la base (6') de ladite pince de trochanter (2') possède une cavité (54) formant un siège pour ledit deuxième joint, vers son extrémité longitudinale, ce siège pouvant loger un appendice (53) de l'organe intermédiaire (4') avec un emboîtement libre et ajusté selon lequel la surface externe dudit appendice (53) étant sensiblement cylindrique avec une ou plusieurs rainures de section radiale sensiblement trapézoïdale.

22. Attèle externe selon la revendication 21, caractérisée en ce qu'une tige de verrouillage (55) est montée entre ledit appendice (53) et ladite cavité (54) et est fixée contre ledit appendice au moyen d'un ou plusieurs boulons de pression (57) vissés sur ladite base (5'), ladite tige ayant une surface interne semi-cylindrique complémentaire de celle dudit appendice (53) afin de réaliser un effet de coin sur ledit appendice.
